(19) Europäisches Patentamt
European Patent Office
Office européen des brevets

(11) Veröffentlichungsnummer: **0 158 138**
**B1**

(12) **EUROPÄISCHE PATENTSCHRIFT**

(45) Veröffentlichungstag der Patentschrift: **12.10.88**

(51) Int. Cl.⁴: **C 07 D 491/048**, A 61 K 31/44

(21) Anmeldenummer: **85102793.8**

(22) Anmeldetag: **12.03.85**

(54) **1-Alkylsubstituierte 1,4-Dihydropyridinlactone, Verfahren zu ihrer Herstellung und ihre Verwendung in Arzneimitteln.**

(30) Priorität: **23.03.84 DE 3410645**

(43) Veröffentlichungstag der Anmeldung: **16.10.85 Patentblatt 85/42**

(45) Bekanntmachung des Hinweises auf die Patenterteilung: **12.10.88 Patentblatt 88/41**

(84) Benannte Vertragsstaaten: **AT BE CH DE FR GB IT LI LU NL SE**

(56) Entgegenhaltungen:
**EP-A-0 083 315**
**DE-A-3 130 041**
**DE-A-3 209 274**
**DE-A-3 209 276**
**US-A-4 284 634**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Goldmann, Siegfried, Dr., Am Osterholz 91, D-5600 Wuppertal 11 (DE)**
Erfinder: **Bossert, Friedrich, Dr., Claudiusweg 7, D-5600 Wuppertal 1 (DE)**
Erfinder: **Bischoff, Hilmar, Dr., Wilkhausstrasse 107, D-5600 Wuppertal 2 (DE)**
Erfinder: **Petzinna, Dieter, Dr., Kölner Landstrasse 80, D-4000 Düsseldorf 13 (DE)**
Erfinder: **Puls, Walter, Dr., In den Birken 75, D-5600 Wuppertal 1 (DE)**
Erfinder: **Schlossmann, Klaus, Dr., In den Beek 116, D-5600 Wuppertal 1 (DE)**

**EP 0 158 138 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

## Beschreibung

Die vorliegende Erfindung betrifft neue 1,4-Dihydropyridinlactone, ein Verfahren zu ihrer Herstellung, sowie ihre Verwendung in Arzneimitteln.

In der EP-A-71 819 sind 4-Aryl-1,4-Dihydropyridinlactone mit positiv inotroper und Blutzucker senkender Wirkung beschrieben.

Die neuen Verbindungen sind durch folgende allgemeine Formel (I) gekennzeichnet:

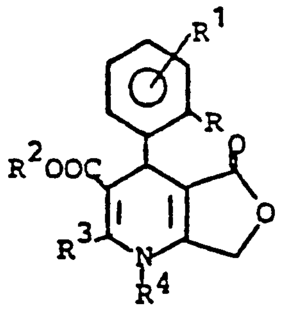

(I)

in welcher

R für Halogen, Alkyl mit 1 bis 10 C-Atomen oder Cyano steht,

$R_1$ für Wasserstoff, Halogen oder $C_1$-$C_{10}$-Alkyl oder $C_1$-$C_{10}$-Alkoxy steht,

$R_2$ für einen $C_1$-$C_{20}$ geradkettigen, verzweigten oder cyclischen, gesättigten oder ungesättigten Kohlenwasserstoffrest steht, der gegebenenfalls durch 1 oder 2 Sauerstoffatome oder -$SO_n$- (n = 0, 1, 2) unterbrochen sein kann und der gegebenenfalls substituiert sein kann durch ein oder mehrere Fluor, Chlor, Brom oder Jodatome, Phenyl, -$NO_2$, -O-$NO_2$, $C_3$-$C_{12}$-Trialkylsilyl, -OH, -CH, .Amino $C_1$-$C_6$-Monoalkylamino, je $C_1$-$C_6$-Dialkylamino Benzyl-$C_1$-$C_6$-alkylamino,

$R^3$ für Wasserstoff, $NH_2$, CHO, CN oder einen $C_1$-$C_8$ geradkettigen oder verzweigten, gesättigten oder ungesättigten Kohlenwasserstoffrest steht, der in der Kette durch Sauerstoff, -NH- oder -N-Alkyl (1 bis 6 C-Atome) unterbrochen sein kann,

$R^4$ für einen $C_1$-$C_{10}$ geradkettigen oder verzweigten Alkyl- oder $C_2$-$C_{10}$-Alkenylrest steht, der gegebenenfalls durch ein oder zwei Sauerstoffatome in der Alkylkette unterbrochen sein kann und der gegebenenfalls substituiert sein kann durch ein bis sechs Fluoratome, Cl, Br, J, CN, $NH_2$, OH, Phenyl, Naphtyl, Hydroxycarbonyl, Alkoxycarbonyl (1 bis 10 C-Atome im Alkoxyrest), -CHO, Morpholino oder $C_1$-$C_4$-Dialkylamino,

in Form von Isomeren, Isomerengemischen, Racematen und optischen Antipoden.

Vorzugsweise seien solche Verbindungen der allgemeinen Formel (I), in welcher

R für Chlor, Fluor, Brom, Alkyl (1 bis 6 C-Atome) oder Cyano steht,

$R^1$ für Wasserstoff, Fluor, Chlor, Alkyl (1 bis 4 C- atome) oder Alkoxy (1 bis 4 C-Atome) steht,

$R^2$ für einen geradkettigen, verzweigten oder cyclischen Alkyl oder Alkenylrest (1 bis 12 C-Atome) steht, der gegebenenfalls durch 1 oder 2 Sauerstoff- oder Schwefelatome oder -SO- unterbrochen sein kann und der gegebenenfalls substituiert sein kann durch Fluor, Phenyl, -$NO_2$, -O-$NO_2$, -OH, Amino, Monoalkylamino (1 bis 3 C-Atome), Cl oder Br,

$R^3$ für Wasserstoff, CN oder einen geradkettigen oder verzweigten Alkyl oder Alkenylrest steht (1 bis 4 C-Atome), der in der Kette durch 0 oder -N(Alkyl)-(1 bis 3 C-Atome) unterbrochen sein kann,

$R^4$ für einen geradkettigen oder verzweigten Alkyl oder Alkenylrest (1 bis 6 C-Atome) steht, der gegebenenfalls durch ein oder zwei Sauerstoffatome in der Alkylkette unterbrochen sein kann und der gegebenenfalls substituiert sein kann durch 1 bis 3 Fluoratome, Cl, $NH_2$, OH, Phenyl, Hydroxycarbonyl, Alkoxycarbonyl (1 bis 3 C-Atome im Alkoxyrest), -CHO oder Morpholino, in Form von Isomeren, Isomerengemischen, Racematen und optischen Antipoden.

Insbesondere seien solche Verbindungen der allgemeinen Formel (I) genannt, in welcher

R für Cyano, Chlor, Fluor, Brom oder $C_1$-$C_3$ Alkyl,

$R_1$ Wasserstoff oder Chlor darstellt,

$R_2$ für $C_1$-$C_6$ Alkyl, welches gegebenenfalls durch 0,S oder -SO- unterbrochen ist, oder für Phenyl-$C_1$-$C_2$-Alkyl steht,

$R_3$ für einen Alkylrest steht (1 bis 3 C-Atome), der in der Kette durch -O- oder $-N(CH_3)-$ unterbrochen sein kann,

$R_4$ $C_1$-$C_3$ Alkyl, $C_2$-$C_3$ Alkylen, oder $C_1$-$C_3$ Alkoxycarbonyl, Hydroxycarbonyl darstellt.

Die erfindungsgemäßen Verbindungen der allgemeinen Formel I können hergestellt werden, indem man

(A) 1,4-Dihydropyridinlactone der allgemeinen Formel II

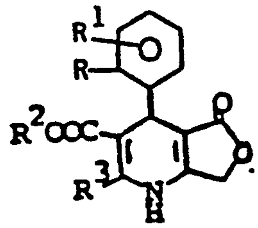

(II)

in welcher R, $R^1$, $R^2$, $R^3$ die oben angegebene Bedeutung haben, in inerten Lösungsmitteln mit Basen deprotoniert und mit Verbindungen der allgemeinen Formel (III)

$R^4$ - X (III)

in welcher $R^4$ die oben angegebene Bedeutung hat und X für eine leicht abspaltbare Gruppe wie Cl, Br, J, $-OSO_2-R^5$ steht, wobei $R^5$ Alkyl oder Aryl bedeutet,
alkyliert,
oder indem man
(B) Aldehyde der allgemeinen Formel IV

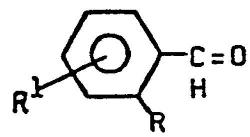

(IV)

in welcher R, $R^1$ die oben angegebene Bedeutung haben, und Amine der allgemeinen Formel V oder deren Salze

$H_2N$ - $R^4$ (V)

in welcher $R^4$ die oben angegebene Bedeutung hat, mit Ketoverbindungen der allgemeinen Formel VI

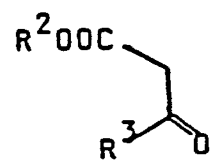

(VI)

in welcher $R^2$, $R^3$ die oben angegebene Bedeutung haben, und Tetronsäure der Formel VII

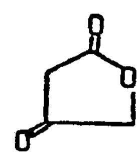

(VII)

gegebenenfalls in Gegenwart von Wasser und/oder inerten organischen Lösungsmitteln umsetzt,
oder indem man
(C) Aldehyde der Formel IV mit Ketoverbindungen der Formel VI und Enaminen der allgemeinen Formel VIII

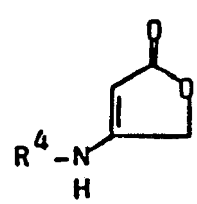

(VIII)

in welcher $R^4$ die oben angegebene Bedeutung hat, gegebenenfalls in Gegenwart von Wasser und/oder inerten organischen Lösungsmitteln umsetzt,
oder indem man

(D) Enamine der allgemeinen Formel IX

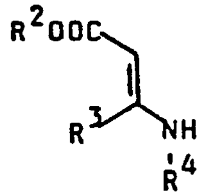

(IX)

in welcher $R^2$, $R^3$, $R^4$ die oben angegebene Bedeutung haben, mit Aldehyden der Formel IV und Tetronsäure der Formel VII gegebenenfalls in Gegenwart von Wasser und/oder inerten organischen Lösungsmitteln umsetzt,

oder indem man

(E) Ylidenverbindungen der allgemeinen Formel X

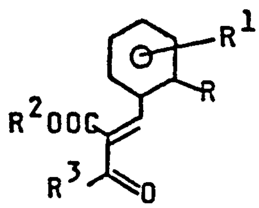

(X)

in welcher $R^1$, $R^2$, $R^3$ die oben angegebene Bedeutung haben mit Enaminen der Formel VIII gegebenenfalls in Gegenwart von Wasser und/oder inerten organischen Lösungsmitteln umsetzt,

oder indem man

(F) Enamine der Formel IX mit Ylidenverbindungen der allgemeinen Formel XI

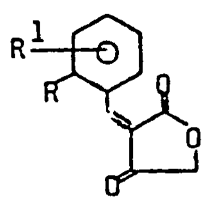

(XI)

in welcher R, $R^1$ die oben angegebene Bedeutung haben, gegebenenfalls in Gegenwart von Wasser oder inerten organischen Lösungsmitteln umsetzt,

oder indem man

(G) Amine der Formel V mit Ylidenverbindungen der Formel X und Tetronsäure der Formel VII gegebenenfalls in Gegenwart von Wasser und/oder inerten organischen Lösungsmitteln umsetzt,

oder indem man

(H) Amine der Formel V mit Ketonverbindungen der Formel VI und Ylidenverbindungen der Formel XI gegebenenfalls in Gegenwart von Wasser und/oder inerten organischen Lösungsmitteln umsetzt,

oder indem man

(I) Dihydropyridine der allgemeinen Formel XII

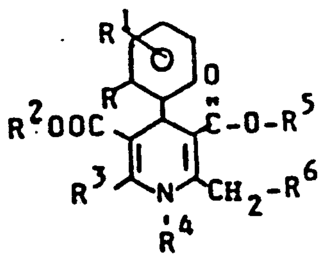

(XII)

in welcher R, $R^1$, $R^2$, $R^3$, $R^4$ die oben angegebene Bedeutung haben,

$R^5$ gleich $R^2$ ist und

$R^6$ für Halogen, bevorzugt Chlor oder Brom steht,

oder

für die Gruppe -O-R[7] steht, wobei R[7] eine übliche Alkohol-Schutzgruppe bedeutet,

gegebenenfalls in Gegenwart eines inerten organischen Lösungsmittels sowie eines geeigneten Hilfsmittels cyclisiert.

Je nach Art der verwendeten Ausgangsverbindungen können nach folgenden Formelschemata hergestellt werden:

(A)

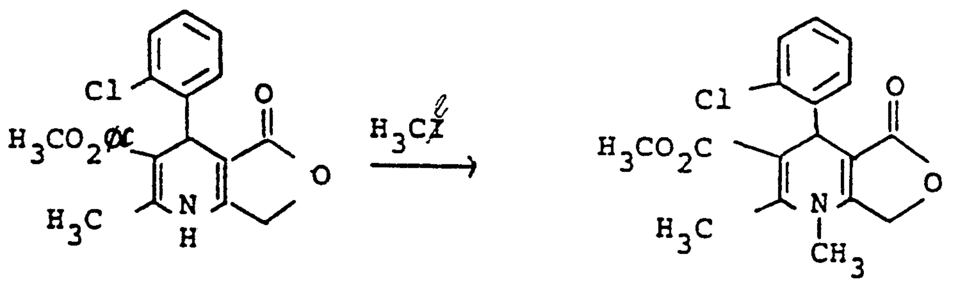


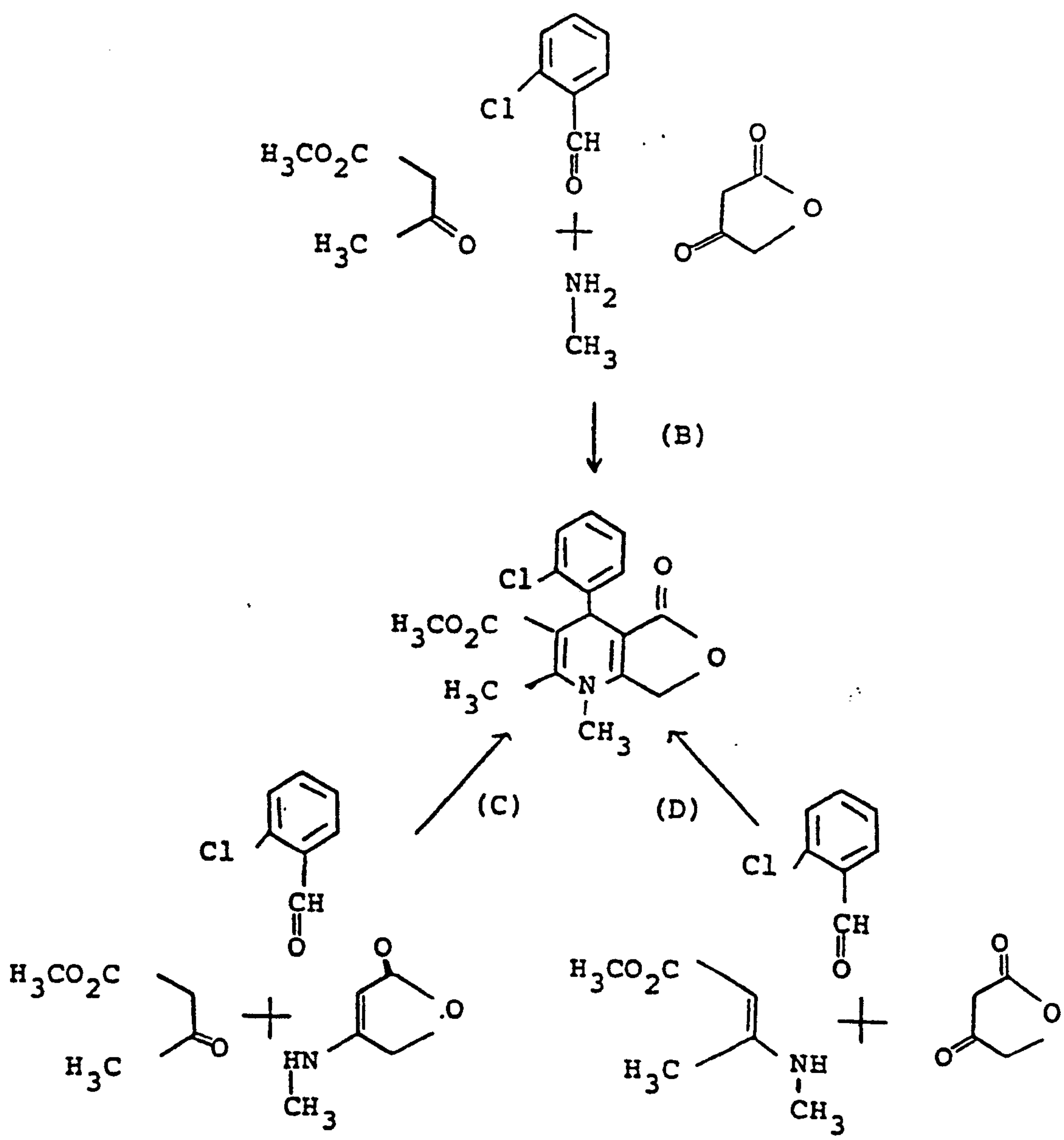

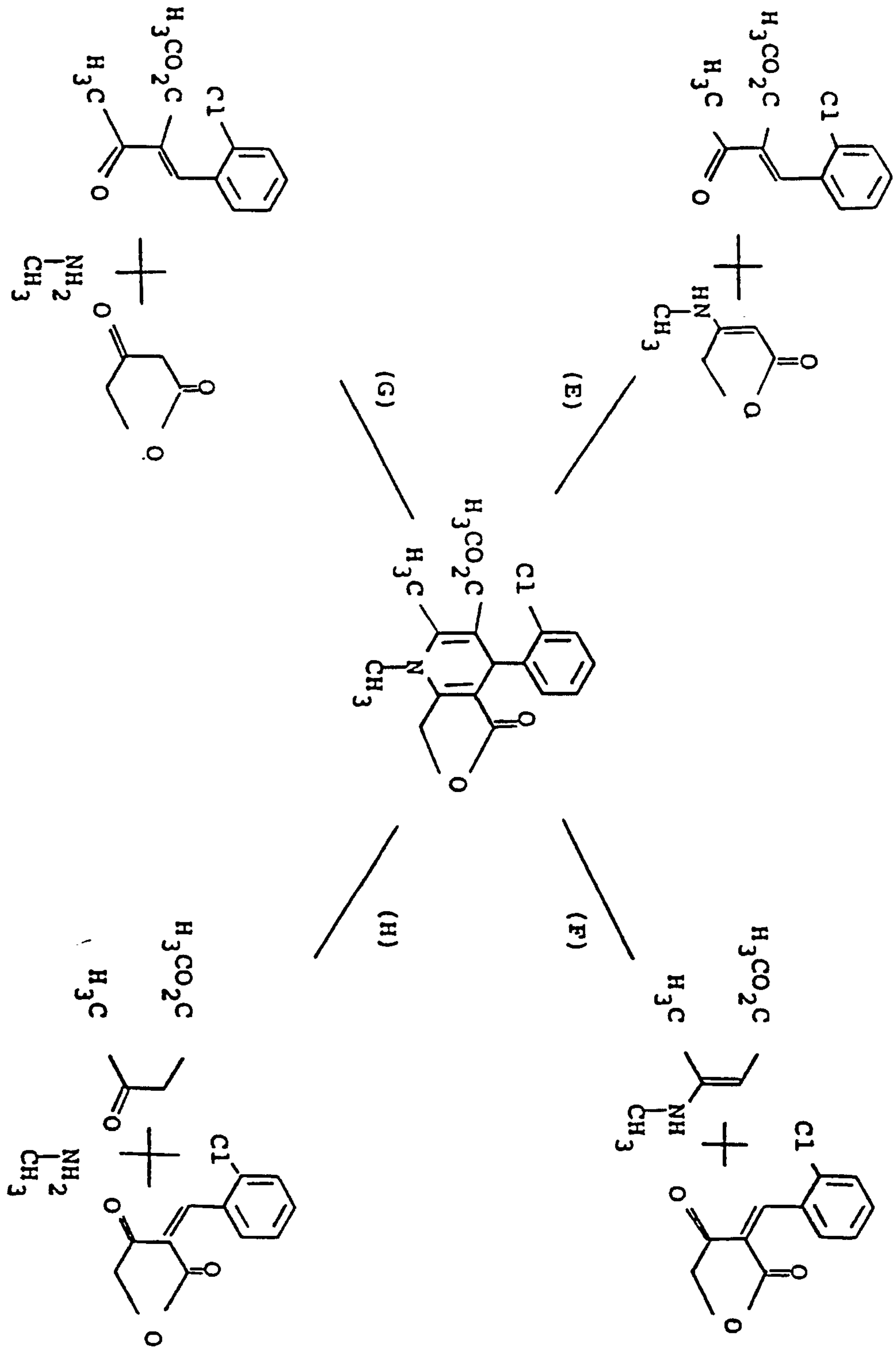


(I)

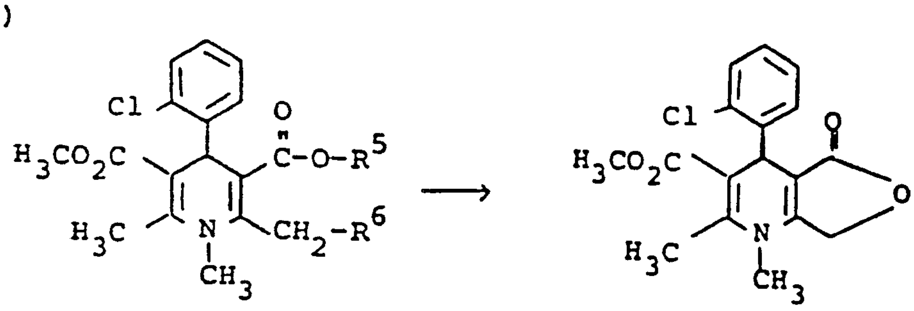

Als Lösungsmittel für Verfahren A kommen alle üblichen inerten Lösungsmittel in Frage. Hierzu gehören vorzugsweise Säureamide wie Dimethylformamid, Hexamethylphosphorsäuretriamid, Ether wie Tetrahydrofuran, Dioxan, Sulfoxide wie Dimethylsulfoxid oder Sulfolan. Als Basen können bei Verfahren A zum Beispiel Metallhydride wie Natriumhydrid, Kaliumhydrid oder Amide wie Natriumamid, 4-Diisopropylamid, Kaliumdiethylamid oder Metallalkyle wie Butyllithium, Phenyllithium oder Hydroxide wie Kaliumhydroxid, Natriumhydroxid oder Alkohole wie Kaliumtert.-butanolat, Kaliummethylat oder Carbonate wie Kaliumcarbonat eingesetzt werden.

Die Alkylierung erfolgt bei Temperaturen von -20°C bis 180°C, vorzugsweise bei Raumtemperatur bis zur Siedetemperatur des verwendeten Lösungsmittels.

Die Alkylierung erfolgt gewöhnlich bei Normaldruck, jedoch kann gegebenenfalls Druck angewendet werden. Die Reaktanden können in beliebigem Mengenverhältnis zueinander eingesetzt werden, bevorzugt wird jedoch ein molares Mengenverhältnis.

Als Lösungsmittel für Verfahren B - H kommen Wasser und alle inerten organischen Lösungsmittel in Frage. Hierzu gehören vorzugsweise Alkohole wie Methanol, Ethanol, Isopropanol, oder Ether wie Diethylether, Dioxan, Tetrahydrofuran, oder Eisessig, Pyridin, Dimethylformamid, Dimethylsulfoxid, Hexamethylphosphorsäuretriamid oder Acetonitril.

Die Verfahren B - I werden bei Temperaturen von 0°C bis 200°C, vorzugsweise von 20°C bis 150°C, durchgeführt.

Normalerweise wird bei Normaldruck gearbeitet, jedoch kann gegebenenfalls auch erhöhter Druck angewendet werden.

Bei der Durchführung der erfindungsgemäßen Verfahren B - H ist das Verhältnis der Reaktanten beliebig. Normalerweise werden die Reaktanten jedoch in molaren Verhältnissen eingesetzt.

Die Cyclisierung (Verfahren I) kann mit oder ohne Lösungsmittel durchgeführt werden. Als Lösungsmittel kommen gegebenenfalls alle üblichen inerten organischen Lösungsmittel in Frage. Hierzu gehören vorzugsweise aromatische Kohlenwasserstoffe wie Benzol, Toluol oder Xylol, Tetralin, Erdölfraktionen, Ether wie Dioxan, Tetrahydrofuran, Glykolmomo- bzw. -diethylether, Halogenkohlenwasserstoffe wie Di-, Tri- oder Tetrachlormethan, Di- oder Trichlorethylen oder Diglyme.

Die Cyclisierung erfolgt in einem Temperaturbereich von 20°C bis 300°C, bevorzugt von 40°C bis 250°C.

Die Cyclisierung kann bei normalem, erhöhtem oder erniedrigtem Druck durchgeführt werden. Im allgemeinen arbeitet man bei Normaldruck.

Als Hilfsmittel bei Verfahren (I) können gegebenenfalls Säuren, Basen, Fluorid oder Wasserstoff eingesetzt werden.

Die Synthese der als Ausgangsstoffe verwendeten 1,4-Dihydropyridinlactone II ist im Europ. Patent 71 819 beschrieben.

Die als Ausgangsstoffe verwendeten Verbindungen der Formel III bis XI sind literaturbekannt oder können nach literaturbekannten Methoden hergestellt werden (vgl. A.C. Cape,.J. Am. Chem. Soc. 67, 1017 (1945). DS-Patent 3 758 515; Organic Reactions XV, 204 ff/ 1907); D. Borrmann, "Umsetzungen von Diketen mit Alkoholen, Phenolen und Mercaptanen in Houben-heyl, Methoden der organischen Chemie," Vol. VII/4, 230 ff (1968); J. Org. Chem. 43, 1541 (1978); Z. Chem. 10, 341 (1907); Suorey at al. J. Am. Chem. Soc. 66, 1933 (1944); DE-OS-3 207 982.

Die Ausgangsverbindungen der allgemeinen Formel XII (Verfahren I), in welcher $R^6$ für die Gruppe -O-$R^7$ steht, sind bekannt oder können nach bekannten Verfahren hergestellt werden. Ausgangsverbindungen der Formel XII mit $R^6$ = Halogen, bevorzugt Chlor, Brom, sind neu und können hergestellt werden, indem man 1,4-Dihydropyridine der allgemeinen Formel XIII

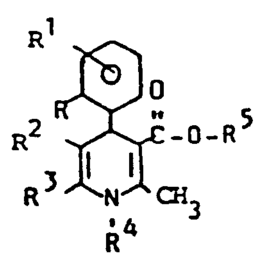

(XIII)

in welher R, $R^1$ - $R^5$ die oben angegebene Bedeutung haben mit Halogenierungsmitteln in Gegenwart von inerten organischen Lösungsmitteln, gegebenenfalls in Anwesenheit von Radikalbildnern umsetzt.

Als Lösungsmittel kommen alle inerten organischen Lösungsmittel in Frage, bevorzugt jedoch halogenierte Kohlenwasserstoffe wie Di-, Tri- oder Tetrachlormethan.

Als Halogenierungsmittel können die üblichen Halogenierungsmittel verwendet werden. Bevorzugt sind Chlor, Brom, N-Chlorsuccinimid oder N-Bromsuccinimid, gegebenenfalls in Anwesenheit von Radikalbildnern wie Azobisisobuttersäurenitril, Dibenzoylperoxid oder Licht.

Die Reaktionstemperaturen können hierbei in einem großen Bereich variiert werden. Im allgemeinen arbeitet man zwischen 0°C und 120°C, bevorzugt bei der Siedetemperatur des verwendeten Lösungsmittels.

Die Umsetzung kann bei normalem Druck, aber auch bei erhöhtem Druck durchgeführt werden. Im allgemeinen arbeitet man bei Normaldruck.

Das Mengenverhältnis der Reaktanden zueinander ist beliebig, bevorzugt werden jedoch äquimolare Mengen eingesetzt.

Die erfindungsgemäßen Verbindungen der allgemeinen Formel (I) zeigen ein wertvolles pharmakologisches Wirkspektrum.

Bei nur geringer Kreislaufwirkung senken sie den Blutzucker und können so zur Behandlung des Diabetes eingesetzt werden.

Die erfindungsgemäßen Verbindungen können in bekannter Weise in die üblichen Formulierungen überführt werden, wie Tabletten, Kapseln, Dragees, Pillen, Granulate, Aerosole, Sirupe, Emulsionen, Suspensionen und Lösungen, unter Verwendung inerter, nichttoxischer, pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d. h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffe, gegebenenfalls unter Verwendung von Emulgiermitteln und/oder Dispergiermittel, wobei z. B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt:

Wasser, nicht-toxische organische Lösungsmittel, wie Paraffine (z. B. Erdölfraktionen), pflanzliche Öle (z. B. Erdnuß-/Sesamöl), Alkohole (z. B. Ethylalkohol, Glycerin), Glykole (z. B. Propylenglykol, Polyethylenglykol), feste Trägerstoffe, wie z. B. natürliche Gesteinsmehle (z. B. Kaoline, Tonerden, Talkum, Kreide), synthetische Gesteinsmehle (z. B. hochdisperse Kieselsäure, Silikate), Zucker (z. B. Rohr-, Milch- und Traubenzucker), Emulgiermitteln (z. B. Polyoxyethylen-Fettsäure-Ester, Polyoxyethylen-Fettalkohol-Ether, Alkylsulfonate und Arylsulfonate), Dispergiermittel (z. B. Lignin, Sulfitablaugen, Methylcellulose, Stärke und Polyvinylpyrrolidon) und Gleitmittel (z. B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen und/oder Elixieren, die für orale Anwendungen gedacht sind, können die Wirkstoffe außer den obengenannten Hilfsstoffen mit verschiedenen Geschmacksaufbessern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei oraler Applikation Mengen von etwa 0,01 bis 200 mg/kg, vorzugsweise 0,1 bis 50 mg/kg Körpergewicht zur Erzielung wirksamer Ergebnisse zu verabreichen.

Trotzdem kann es gegebenenfalls erforderlich sein, von den genannten Mengen abzuweichen, und zwar in Abhängigkeit vom Körpergewicht des Versuchstieres bzw. der Art des Applikationsweges, aber auch aufgrund der Tierart und deren individuellem Verhalten gegenüber dem Medikament bzw. der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäß gelten hierbei auch die obigen Ausführungen.

**Beispiel 1**

4-(2-Chlorphenyl)-1-ethyl-2-methyl-5-oxo-1,4,5,7-tetrahydrofuro-[3,4-b]pyridin-3-carbonsäure-ethylester

50 mmol 4-(2-Chlorphenyl)-2-methyl-5-oxo-1,4,5,7-tetrahydrofuro-[3,4-b]pyridin-3-carbonsäure-ethylester werden in Tetrahydrofuran gelöst, bei -78°C mit 50 mmol Lithiumdiisopropylamid und anschließend mit 50 mmol Ethyljodid versetzt. Es wird auf Raumtemperatur erwärmt und 1h gerührt. Nach Einengen wird mit $CH_2Cl_2$ aufgenommen, mit Wasser gewaschen, getrocknet, eingeengt und umkristallisiert.

Fp: 140 - 141°C.

**Beispiel 2**

4-(2-Chlorphenyl)-1,2-dimethyl-5-oxo-1,4,5,7-tetrahydrofuro-[3,4-b]pyridin-3-carbonsäurepropylester

50 mmol 4-(2-Chlorphenyl)-2-methyl-5-oxo-1,4,5,7-tetrahydrofuro-[3,4-b]pyridin-3-carbonsäurepropylester werden in 150 ml Dimethylsulfoxid gelöst, mit 7 g Kaliumhydroxidpulver und 50 mmol Methyljodid versetzt. Nach 2h bei Raumtemperatur wird auf Eiswasser gegossen, mit $CH_2Cl_2$ extrahiert, getrocknet, eingeengt und umkristallisiert.

Fp: 173 - 177°C.

Analog werden dargestellt:

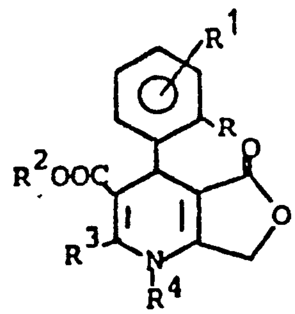

| Beispiel | R | $R^1$ | $R^2$ | $R^3$ | $R^4$ | FP |
|---|---|---|---|---|---|---|
| 3 | -Cl | H | $-C_2H_5$ | $-CH_3$ | $-CH_2-CH=CH_2$ | 132° |
| 4 | -Cl | H | $-CH_3$ | $-CH_3$ | $-CH_2-CH_3$ | 137 - 9° |
| 5 | -Cl | H | $-(CH_2)_3-CH_3$ | $-CH_3$ | $-CH_2-CH_3$ | 169 - 74° |
| 6 | -Cl | H | $-CH_2-CH_3$ | $-CH_3$ | $-(CH_2)_2-CH_3$ | 136 - 9° |
| 7 | -Cl | H | $-CH(CH_3)_2$ | $-CH_3$ | $-CH_2-CH_3$ | 110 - 12° |
| 8 | -Cl | H | $-(CH_2)_2-OCH_3$ | $-CH_3$ | $-CH_2-CH_3$ | 145° |
| 9 | -Cl | H | $-(CH_2)_2-S-CH_3$ | $-CH_3$ | $-CH_2-CH_3$ | 131 - 35° |
| 10 | -Cl | $H_3H$ | $-C_2H_5$ | $-CH_3$ | $-C_2H_5$ | 103 - 10° |
| 11 | -Cl | H | $-(CH_2)_2-CH_3$ | $-CH_3$ | $-C_2H_5$ | 146 - 50° |
| 12 | -Cl | 3-Cl | $-C_2H_5$ | $-CH_3$ | $-C_2-H_5$ | 123 - 5° |
| 13 | -Cl | H | $-(CH_2)_2-$ 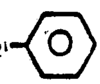 | $-CH_3$ | $-C_2H_5$ | 87° |
| 14 | -Cl | 6-Cl | $-C_2H_5$ | $-CH_3$ | $-C_2H_5$ | 130° |
| 15 | -Cl | 4-Cl | $-C_2H_5$ | $-CH_3$ | $-C_2H_5$ | 121 - 4° |
| 16 | -Cl | H | $-(CH_2)_2-\overset{O}{\overset{\|\|}{S}}-CH_3$ | $-CH_3$ | $-C_2H_5$ | amorph 1) |
| 17 | -Cl | H | $-(CH_2)_5-CH_3$ | $-CH_3$ | $-C_2H_5$ | Öl |
| 18 | -F | H | $-C_2H_5$ | $-CH_3$ | $-C_2H_5$ | 183 - 6° |
| 19 | -Br | H | $-C_2H_5$ | $-CH_3$ | $-C_2H_5$ | 141 - 3° |
| 20 | -Cl | H | $-C_2H_5$ | $-CH_3$ | $-CH_2-COOH$ | 135 - 139° |
| 21 | -Cl | H | $-C_2H_5$ | $-CH_3$ | $-CH_2-COOC_2H_5$ | amorph |
| 22 | -Cl | H | $-C_2H_5$ | $-CH_2-OCH_3$ | $-CH_3$ | 134 - 7° |
| 23 | -Cl | H | $-C_2H_5$ | $-CH_2-CH_2-N(CH_3)_2$ | $-C_2H_5$ | 144 - 7° |
| 24 | -Cl | H | $-(CH_2)_2-OC_2H_5$ | $-CH_3$ | $-CH_3$ | 147 - 9° |
| 25 | -Cl | H | $-(CH_2)_2-OC_2H_5$ | $-CH_3$ | $-CH_2-CH_3$ | 120 - 2° |
| 26 | $-CH_3$ | H | $-(CH_2)_2-OCH_3$ | $-CH_3$ | $-CH_2-CH_3$ | 151 - 4° |
| 27 | -Cl | H | $-(CH_2)_2-OCH_3$ | $-CH_3$ | $-CH_3$ | 171 - 3° |
| 28 | -Cl | H | $-(CH_2)_2-OCH_3$ | $-CH_3$ | $-CH_2-CH=CH_2$ | 99 - 100° |
| 29 | -Cl | H | $-(CH_2)_2-O-(CH_2)_2-OCH_3$ | $-CH_3$ | $-CH_3$ | 115 - 7° |
| 30 | -Cl | H | $-C(CH_3)_3$ | $-CH_3$ | $-CH_2-CH_3$ | 131 - 3° |
| 31 | -Cl | H | $-(CH_2)_2-CH_3$ | $-CH_3$ | $-CH_2-CH_3$ | 133 - 5° |
| 32 | $-CH_3$ | H | $-CH(CH_3)_2$ | $-CH_3$ | $-CH_2-CH_3$ | 100 - 3° |
| 33 | -CN | H | $-CH(CH_3)_2$ | $-CH_3$ | $-CH_2-CH_3$ | 152 - 5° |

1) 423 (M+), 406 (90 %), 378, 332, 316, 312, 288, 248, 222, 177, 75

Die blutglucosesenkende Wirkung der zu untersuchenden Substanzen wurde an männlichen Wistar-Ratten mit einem Gewicht zwischen 140 und 190 g geprüft. Die Ratten wurden zu diesem Zweck 18 h vor der

Applikation der Substanzen gewogen, in Gruppen zu 6 Tieren eingeteilt und nüchtern gesetzt. Die zu untersuchenden Substanzen wurden direkt vor der Applikation in wässriger 0,75 %-iger Traganthsuspension mit einem Ultra-Turrax suspendiert. Die Applikation der Traganthsuspension (Kontrolltiere) bzw. der in Traganth suspendierten Substanzen erfolgte mittels einer Schlundsonde.

Die Blutentnahme erfolgte bei jeder Ratte 30, 60 und 120 min nach der Applikation aus dem retroorbitalen Venenplexus. Jeweils 30 µl Blut wurden mit einem automatischen Dilutor entnommen und mit 0,3 ml Uranylacetat (0,16 %) enteiweißt. Nach der Zentrifugation wurde die Glucose im Überstand nach der Glucoseoxidase-Methode mit 4-Amino-phenazon als Farbreagenz an einem Gemsaec Fastanalyzer photometrisch bestimmt. Die Auswertung der Ergebnisse erfolgte mit dem t-Test nach Student, als Signifikanzgrenze wurde $p < 0{,}05$ gewählt.

Substanzen, die bei Ratten zu einem Zeitpunkt eine signifikante Reduktion der Blutglucosekonzentration um mindestens 10 % bewirkten, verglichen mit der Kontrollgruppe, die nur Traganthsuspension erhielt, wurden als wirksam angegeben.

Die nachfolgende Tabelle 1 enthält die gefundenen Veränderungen der Blutglucosekonzentrationen in Prozent der Kontrolle.

**Tabelle 1**

| Substanz (Patentbeispiel Nr.) | Abnahme der Blutglucose-konzentration in % der Kontrolle 30 mg/kg p.o. |
|---|---|
| 1 | 23 |
| 3 | 19 |
| 8 | 26 |
| 10 | 19 |
| 15 | 28 |

**Patentansprüche**

1. Dihydropyridinlactone der allgemeinen Formel I

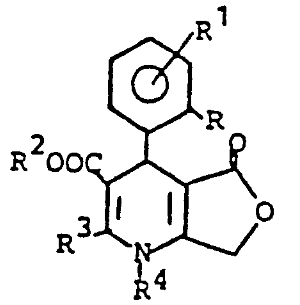

(I)

in welcher

R für Halogen, Alkyl mit 1 bis 10 C-Atomen oder Cyano steht,

$R_1$ für Wasserstoff, Halogen oder $C_1$-$C_{10}$-Alkyl oder $C_1$-$C_{10}$-Alkoxy steht,

$R_2$ für einen $C_1$-$C_{20}$ geradkettigen, verzweigten oder cyclischen, gesättigten oder ungesättigten Kohlenwasserstoffrest steht, der gegebenenfalls durch 1 oder 2 Sauerstoffatome oder $-SO_n-$ (n = 0, 1, 2) unterbrochen sein kann und der gegebenenfalls substituiert sein kann durch ein oder mehrere Fluor, Chlor, Brom oder Jodatome, Phenyl, $-NO_2$, $-O-NO_2$, $C_3$-$C_{12}$-Trialkylsilyl, -OH, -CN, Amino, $C_1$-$C_6$-Monoalkylamino, je $C_1$-$C_6$-Dialkylamino, Benzyl-$C_1$-$C_6$-alkylamino,

$R^3$ für Wasserstoff, $NH_2$, CHO, CN oder einen $C_1$-$C_8$ geradkettigen oder verzweigten, gesättigten oder ungesättigten Kohlenwasserstoffrest steht, der in der Kette durch Sauerstoff, -NH- oder -N-Alkyl (1 bis 6 C-Atome) unterbrochen sein kann,

$R^4$ für einen $C_1$-$C_{10}$ geradkettigen oder verzweigten Alkyl- oder $C_2$-$C_{10}$-Alkenylrest steht, der gegebenenfalls durch ein oder zwei Sauerstoffatome in der Alkylkette unterbrochen sein kann und der gegebenenfalls substituiert sein kann durch ein bis sechs Fluoratome, Cl, Br, J, CN, $NH_2$, OH, Phenyl, Napthyl, Hydroxycarbonyl, Alkoxycarbonyl (1 bis 10 C-Atome im Alkoxyrest), -CHO, Morpholino oder $C_1$-

$C_4$-Dialkylamino,
in Form von Isomeren, Isomerengemischen, Racematen und optischen Antipoden.

2. Dihydropyridinlactone der allgemeinen Formel I in Anspruch 1, in der

R für Chlor, Fluor, Brom, Alkyl (1 bis 6 C-Atome) oder Cyano steht,

$R^1$ für Wasserstoff, Fluor, Chlor, Alkyl (1 bis 4 C- atome) oder Alkoxy (1 bis 4 C-Atome) steht,

$R^2$ für einen geradkettigen, verzweigten oder cyclischen Alkyl oder Alkenylrest (1 bis 12 C-Atome) steht, der gegebenenfalls durch 1 oder 2 Sauerstoff- oder Schwefelatome oder -SO- unterbrochen sein kann und der gegebenenfalls substituiert sein kann durch Fluor, Phenyl, $-NO_2$, $-O-NO_2$, -OH, Amino, Monoalkylamino (1 bis 3 C-Atome), Cl oder Br,

$R^2$ für Wasserstoff, CN, oder einen geradkettigen oder verzweigten Alkyl oder Alkenylrest steht (1 bis 4 C-Atome), der in der Kette durch 0 oder -N(Alkyl)-(1 bis 3 C-Atome) unterbrochen sein kann,

$R^4$ für einen geradkettigen oder verzweigten Alkyl oder Alkenylrest (1 bis 6 C-Atome) steht, der gegebenenfalls durch ein oder zwei Sauerstoffatome in der Alkylkette unterbrochen sein kann und der gegebenenfalls substituiert sein kann durch 1 bis 3 Fluoratome, Cl, $NH_2$, OH, Phenyl, Hydroxycarbonyl, Alkoxycarbonyl (1 bis 3 C-Atome im Alkoxyrest), -CHO oder Morpholino, in Form von Isomeren, Isomerengemischen, Racematen und optischen Antipoden.

3. Dihydropyridinlactone der allgemeinen Formel I in Anspruch 1, in der

R für Chlor, Fluor, Brom, $C_1$-$C_3$-Alkyl, steht,

$R_1$ Wasserstoff oder Chlor darstellt,

$R_2$ für $C_1$-$C_6$-Alkyl, welches gegebenenfalls durch O, S oder SO unterbrochen ist, oder für Phenyl-$C_1$-$C_2$-Alkyl steht,

$R^3$ für einen $C_1$-$C_3$-Alkylrest steht, der in der Kette durch -O- oder -N- unterbrochen sein kann,
$CH_3$

$R^4$ $C_1$-$C_3$-Alkyl, $C_2$-$C_3$-Alkylen, gegebenenfalls substituiert durch $C_1$-$C_3$-Alkoxycarbonyl, Hydroxycarbonyl darstellt.

4. Dihydropyridinlactone der Ansprüche 1 - 3 zur Bekämpfung von Erkrankungen.

5. Dihydropyridinlactone der Ansprüche 1 - 3 zur Bekämpfung von Diabetes.

6. Verfahren zur Herstellung der Dihydropyridinlactone der Formel I

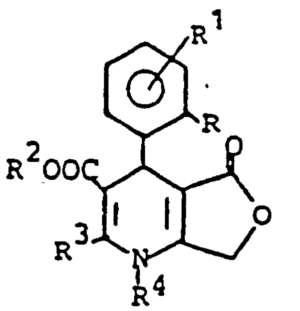

(I)

in welcher

R für Halogen mit 1 bis 10 C-Atomen oder Cyano steht,

$R_1$ für Wasserstoff, Halogen oder $C_1$-$C_{10}$-Alkyl oder $C_1$-$C_{10}$-Alkoxy steht,

$R_2$ für einen $C_1$-$C_{20}$ geradkettigen, verzweigten oder cyclischen, gesättigten oder ungesättigten Kohlenwasserstoffrest steht, der gegebenenfalls durch 1 oder 2 Sauerstoffatome oder $-SO_n$- (n = 0, 1, 2) unterbrochen sein kann und der gegebenenfalls substituiert sein kann durch ein oder mehrere Fluor, Chlor, Brom oder Jodatome, Phenyl, $-NO_2$-, $-O-NO_2$, $C_3$-$C_{12}$-Trialkylsilyl, -OH, -CN, Amino, $C_1$-$C_6$-Monoalkylamino, je $C_1$-$C_6$-Dialkylamino, Benzyl-$C_1$-$C_6$-alkylamino,

$R^3$ für Wasserstoff, $NH_2$, CHO, CN oder einen $C_1$-$C_8$ geradkettigen oder verzweigten, gesättigten oder ungesättigten Kohlenwasserstoffrest steht, der in der Kette durch Sauerstoff, -NH- oder -N-Alkyl (1 bis 6 C-Atome) unterbrochen sein kann,

$R^4$ für einen $C_1$-$C_{10}$ geradkettigen oder verzweigten Alkyl- oder $C_2$-$C_{10}$-Alkenylrest steht, der gegebenenfalls durch ein oder zwei Sauerstoffatome in der Alkylkette unterbrochen sein kann und der gegebenenfalls substituiert sein kann durch ein bis sechs Fluoratome, Cl, Br, J, CN, $NH_2$, OH, Aryl, Hydroxycarbonyl, Alkoxycarbonyl (1 bis 10 C-Atome im Alkoxyrest), -CHO, Morpholino oder $C_1$-$C_4$-Dialkylamino,

dadurch gekennzeichnet daß man

(A) 1,4-Dihydropyridinlactone der allgemeinen Formel II

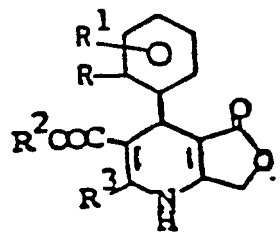

(II),

in welcher R, $R^1$, $R^2$, $R^3$ die oben angegebene Bedeutung haben, in inerten Lösungsmitteln mit Basen deprotoniert und mit Verbindungen der allgemeinen Formel III

$R^4$ - X (III),

in welcher $R^4$ die oben angegebene Bedeutung hat und
X - für eine leicht abspaltbare Gruppe wie Cl, Br, J, $-OSO_2-R^5$ steht, wobei $R^5$ - Alkyl oder Aryl bedeutet,

alkyliert,
oder daß man
(B) Aldehyde der allgemeinen Formel IV

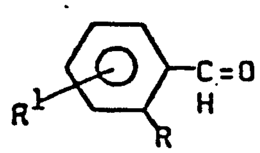

(IV),

in welcher R, $R^1$ die oben angegebene Bedeutung haben, und Amine der allgemeinen Formel V oder deren Salze

$H_2N-R^4$ (V),

in welcher $R^4$ die oben angegebene Bedeutung hat, mit Ketoverbindungen der allgemeinen Formel VI

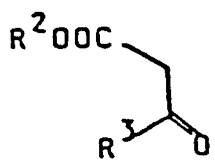

(VI)

in welcher $R^2$, $R^3$ die oben angegebene Bedeutung haben, und Tetronsäure der Formel VII

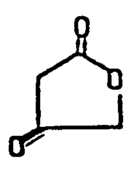

(VII)

gegebenenfalls in Gegenwart von Wasser und/oder inerten organischen Lösungsmitteln umsetzt,
oder daß man
(C) Aldehyde der Formel IV mit Ketoverbindungen der Formel VI und Enaminen der allgemeinen Formel VIII

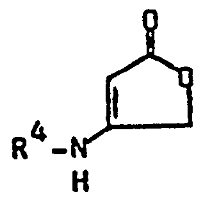

(VIII)

in welcher $R^4$ die oben angegebene Bedeutung hat, gegebenenfalls in Gegenwart von Wasser und/oder inerten organischen Lösungsmitteln umsetzt,

oder daß man

(D) Enamine der allgemeinen Formel IX

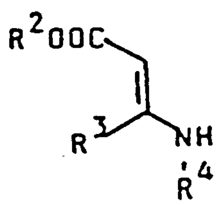

(IX),

in welcher $R^2$, $R^3$, $R^4$ die oben angegebene Bedeutung haben, mit Aldehyden der Formel IV und Tetronsäure der Formel VII, gegebenenfalls in Gegenwart von Wasser und/oder inerten organischen Lösungsmitteln umsetzt,

oder daß man

(E) Ylidenverbindungen der allgemeinen Formel X

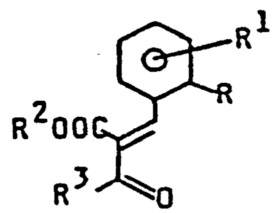

(X),

in welcher $R^1$, $R^2$, $R^3$ die oben angegebene Bedeutung haben, mit Enaminen der Formel VIII gegebenenfalls in Gegenwart von Wasser und/oder inerten organischen Lösungsmitteln umsetzt,

oder daß man

(F) Enamine der Formel IX mit Ylidenverbindungen der allgemeinen Formel XI

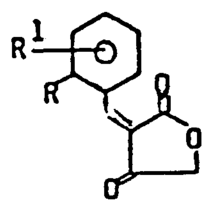

(XI),

in welcher R, $R^1$ die oben angegebene Bedeutung haben, gegebenenfalls in Gegenwart von Wasser oder inerten organischen Lösungsmitteln umsetzt,

oder daß man

(G) Amine der Formel V mit Ylidenverbindungen der Formel X und Tetronsäure der Formel VII gegebenenfalls im Gegenwart von Wasser und/oder inerten organischen Lösungsmitteln umsetzt,

oder daß man

(H) Amine der Formel V mit Ketoverbindungen der Formel VI und Ylidenverbindungen der Formel XI gegebenenfalls in Gegenwart von Wasser und/ oder inerten organischen Lösungsmitteln umsetzt,

oder daß man

(I) Dihydropyridine der allgemeinen Formel XII

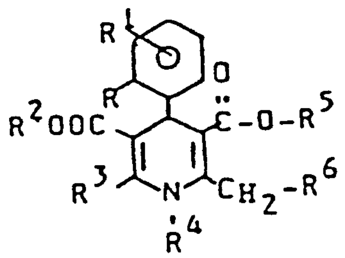

(XII),

in welcher R, $R^1$, $R^2$, $R^3$, $R^4$ die oben angegebene Bedeutung haben, $R^5$ die gleiche Bedeutung wie $R^2$ hat,
$R^6$ - für Halogen steht, oder
- für die Gruppe $O$-$R^7$ steht, wobei $R^7$ eine übliche Alkohol-Schutzgruppe bedeutet,

gegebenenfalls in Gegenwart eines inerten organischen Lösungsmittels sowie eines geeigneten Hilfsmittels cyclisiert.

7. Verfahren gemäß Anspruch 6 zur Herstellung von Dihydropyridinlactonen der Formel I in Anspruch 6, in der
$R$ für Chlor, Fluor, Brom, Cyano oder $C_1$-$C_3$-Alkyl steht,
$R_1$ Wasserstoff oder Chlor darstellt,
$R_2$ für $C_1$-$C_6$-Alkyl, welches gegebenenfalls durch O, S oder SO unterbrochen ist, oder für Phenyl-$C_1$-$C_2$-Alkyl steht,
$R_3$ für einen $C_1$-$C_3$-Alkylrest steht, der in der Kette durch -O- oder -N- unterbrochen sein kann,

$CH_3$

$R_4$ $C_1$-$C_3$-Alkyl, $C_2$-$C_3$-Alkylen, gegebenenfalls substituiert durch $C_1$-$C_3$-Alkoxycarbonyl, Hydroxycarbonyl darstellt.

8. Verfahren gemäß Anspruch 6 zur Herstellung von 4-(2-Chlorphenyl)-1-ethyl-2-methyl-5-oxo-1,4,5,7-tetrahydrofuro[3,4-b]-pyridin-3-carbonsäureisopropylester.

9. Arzneimittel enthaltend mindestens eine Verbindung der allgemeinen Formel I gemäß Anspruch 1.

10. Verwendung der Dihydropyridinlactone gemäß Ansprüchen 1 bis 3 zur Herstellung von Arzneimitteln zur Bekämpfung von Diabetes.

## Claims

1. Dihydropyridinelactones of the general formula I

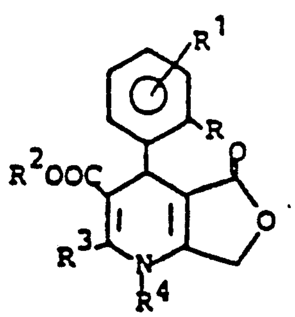

(I)

in which
$R$ represents halogen, alkyl having 1 to 10 C atoms or cyano,
$R^1$ represents hydrogen, halogen or $C_1$-$C_{10}$-alkyl or $C_1$-$C_{10}$-alkoxy,
$R^2$ represents a $C_1$-$C_{20}$ straight-chain, branched or cyclic, saturated or unsaturated hydrocarbon radical which can optionally be interrupted by 1 or 2 oxygen atoms or $-SO_n-$ ($n = 0$, 1 or 2) and which can optionally be substituted by one or more fluorine, chlorine, bromine or iodine atoms, phenyl, $-NO_2$, $-O-NO_2$, $C_3$-$C_{12}$-trialkylsilyl, -OH, -CN, amino, $C_1$-$C_6$-monoalkylamino, dialkylamino in which each alkyl is $C_1$-

$C_6$, or benzyl-$C_1$-$C_6$-alkyl-amino,

$R^3$ represents hydrogen, $NH_2$, CHO, CN or a $C_1$-$C_8$ straight-chain or branched, saturated or unsaturated hydrocarbon radical which can be interrupted in the chain by oxygen, -NH- or -N-alkyl (1 to 6 C atoms), and

$R^4$ represents a $C_1$-$C_{10}$ straight-chain or branched alkyl or $C_2$-$C_{10}$-alkenyl radical which can optionally be interrupted by one or two oxygen atoms in the alkyl chain and which can optionally be substituted by one to six fluorine atoms, Cl, Br, I, CN, $NH_2$, OH, phenyl, naphthyl, hydroxycarbonyl, alkoxycarbonyl (1 to 10 C atoms in the alkoxy radical), -CHO, morpholino or $C_1$-$C_4$-dialkylamino,

in the form of isomers, isomer mixtures, racemates and optical antipodes.

2. Dihydropyridinelactones of the general formula I in Claim 1,
in which

R represents chlorine, fluorine, bromine, alkyl, (1 to 6 C atoms) or cyano,

$R^1$ represents hydrogen, fluorine, chlorine, alkyl, (1 to 4 C atoms) or alkoxy (1 to 4 C atoms),

$R^2$ represents a straight-chain, branched or cyclic alkyl or alkenyl radical (1 to 12 C atoms) which can optionally be interrupted by 1 or 2 oxygen or sulphur atoms or -SO- and which can optionally be substituted by fluorine, phenyl, $-NO_2$, $-O-NO_2$, -OH, amino, monoalkylamino (1 to 3 C atoms), Cl or Br,

$R^3$ represents hydrogen, CN or a straight-chain or branched alkyl or alkenyl radical (1 to 4 C atoms) which can be interrupted in the chain by O or -N(alkyl)- (1 to 3 C atoms), and

$R^4$ represents a straight-chain or branched alkyl or alkenyl radical (1 to 6 C atoms) which can optionally be interrupted by one or two oxygen atoms in the alkyl chain and which can optionally be substituted by 1 to 3 fluorine atoms, Cl, $NH_2$, OH, phenyl, hydroxycarbonyl, alkoxycarbonyl (1 to 3 C atoms in the alkoxy radical), -CHO or morpholino,

in the form of isomers, isomer mixtures, racemates and optical antipodes.

3. Dihydropyridinelactones of the general fomula I in Claim 1,
in which

R represents chlorine, fluorine, bromine or $C_1$-$C_3$-alkyl,

$R^1$ represents hydrogen or chlorine,

$R^2$ represents $C_1$-$C_6$-alkyl which is optionally interrupted by O, S or SO, or represents phenyl-$C_1$-$C_2$-alkyl,

$R^3$ represents a $C_1$-$C_3$-alkyl radical which can be interrupted in the chain by -O- or $-\underset{CH_3}{\underset{|}{N}}-$, and

$R^4$ represents $C_1$-$C_3$-alkyl, $C_2$-$C_3$-alkylene, optionally substituted by $C_1$-$C_3$-alkoxycarbonyl, or hydroxycarbonyl.

4. Dihydropyridinelactones of Claims 1 - 3 for combating diseases.

5. Dihydropyridinelactones of Claims 1 - 3 for combating diabetes.

6. Process for the preparation of dihydropyridinelactones of the formula I

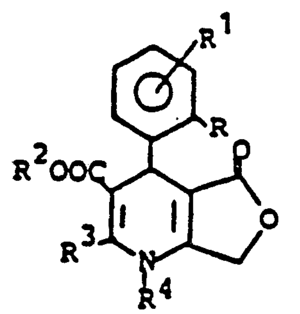

(I)

in which

R represents halogen, alkyl having 1 to 10 C atoms or cyano,

$R^1$ represents hydrogen, halogen or $C_1$-$C_{10}$-alkyl or $C_1$-$C_{10}$-alkoxy,

$R^2$ represents a $C_1$-$C_{20}$ straight-chain, branched or cyclic, saturated or unsaturated hydrocarbon radical which can optionally be interrupted by 1 or 2 oxygen atoms or $-SO_n-$ (n = 0, 1 or 2) and which can optionally be substituted by one or more fluorine, chlorine, bromine or iodine atoms, phenyl, $-NO_2$, $-O-NO_2$, $C_3$-$C_{12}$-trialkylsilyl, -OH, -CN, amino, $C_1$-$C_6$-monoalkylamino, dialkylamino in which each alkyl is $C_1$-$C_6$, or benzyl-$C_1$-$C_6$-alkyl-amino,

$R^3$ represents hydrogen, $NH_2$, CHO, CN or a $C_1$-$C_8$ straight-chain or branched, saturated or unsaturated hydrocarbon radical which can be interrupted in the chain by oxygen, -NH- or -N-alkyl (1 to 6 C atoms),

and

$R^4$ represents a $C_1$-$C_{10}$ straight-chain or branched alkyl or $C_2$-$C_{10}$-alkenyl radical which can optionally be interrupted by one or two oxygen atoms in the alkyl chain and which can optionally be substituted by one to six fluorine atoms, Cl, Br, I, CN, $NH_2$, OH, aryl, hydroxycarbonyl, alkoxycarbonyl (1 to 10 C atoms in the alkoxy radical), -CHO, morpholino or $C_1$-$C_4$-dialkylamino,

characterized in that
(A) 1,4-dihydropyridinelactones of the general formula II

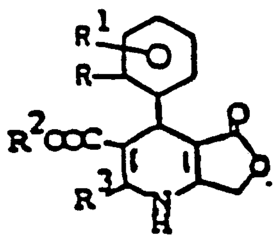

(II)

in which
R, $R^1$, $R^2$ and $R^3$ have the abovementioned meaning
are deprotonated in inert solvents using bases and are alkylated with compounds of the general formula III
in which

$R^4$ has the abovementioned meaning and

X represents a group which can easily be split off, such as Cl, Br, I, $-OSO_2-R^5$,

where

$R^5$ denotes alkyl or aryl,

or in that
(B) aldehydes of the general formula IV

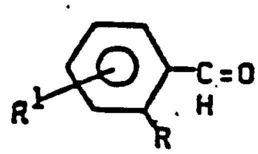

(IV)

in which
R and $R^1$ have the abovementioned meaning, and amines of the general formula V or salts thereof

$H_2N-R^4$ (V)

in which
$R^4$ has the abovementioned meaning are reacted with keto compounds of the general formula VI

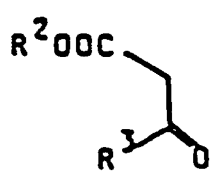

(VI)

in which
$R^2$ and $R^3$ have the abovementioned meaning and tetronic acid of the formula VII

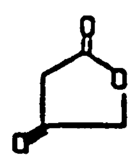

(VII)

if appropriate in the presence of water and/or inert organic solvents,
or in that
(C) aldehydes of the formula IV are reacted with keto compounds of the formula VI and enamines of the general formula VIII

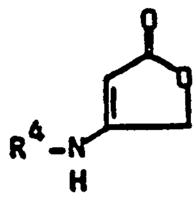

(VIII)

in which
$R^4$ has the abovementioned meaning, if appropriate in the presence of water and/or inert organic solvents, or in that
(D) enamines of the general formula IX

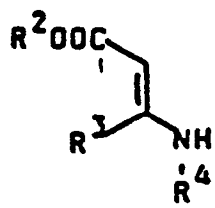

(IX)

in which
$R^2$, $R^3$ and $R^4$ have the abovementioned meaning, are reacted with aldehydes of the formula IV and tetronic acid of the formula VII, if appropriate in the presence of water and/or inert organic solvents, or in that
(E) ylidene compounds of the general formula X

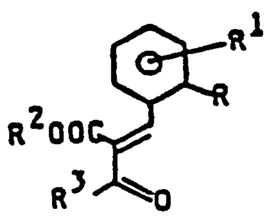

(X)

in which
$R^1$, $R^2$ and $R^3$ have the abovementioned meaning, are reacted with enamines of the formula VIII, if appropriate in the presence of water and/or inert organic solvents, or in that
(F) enamines of the formula IX are reacted with ylidene compounds of the general formula XI

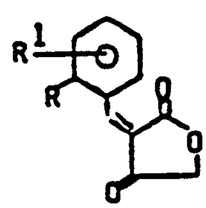

(XI)

in which
R and $R^1$ have the abovementioned meaning, if appropriate in the presence of water or inert organic solvents,
or in that
(G) amines of the formula V are reacted with ylidene compounds of the formula X and tetronic acid of the formula VII, if appropriate in the presence of water and/or inert organic solvents,
or in that
(H) amines of the formula V are reacted with keto compounds of the formula VI and ylidene compounds of the formula XI, if appropriate in the presence of water and/or inert organic solvents,
or in that

(l) dihydropyridines of the general formula XII

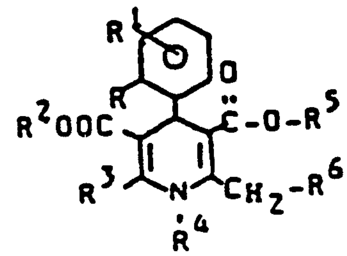

(XII)

in which
R, $R^1$, $R^2$, $R^3$ and $R^4$ have the abovementioned meaning,
$R^5$ has the same meaning as $R^2$, and
$R^6$ represents halogen, or represents the group O-R,
where
$R^7$ denotes a customary alcohol protective group,
are cyclized, if appropriate in the presence of an inert organic solvent and a suitable auxiliary.

7. Process according to Claim 6 for the preparation of dihydropyridinelactones of the formula I in Claim 6, in which
R represents chlorine, fluorine, bromine, cyano or $C_1$-$C_3$-alkyl,
$R^1$ represents hydrogen or chlorine,
$R^2$ represents $C_1$-$C_6$-alkyl which is optionally interrupted by O, S or SO, or represents phenyl-$C_1$-$C_2$-alkyl,
$R^3$ represents a $C_1$-$C_3$-alkyl radical which can be interrupted in the chain by -O- or -N(CH$_3$)-
$R^4$ represents $C_1$-$C_3$-alkyl, $C_2$-$C_3$-alkylene, optionally substituted by $C_1$-$C_3$-alkoxycarbonyl, or hydroxycarbonyl.

8. Process according to Claim 6 for the preparation of isopropyl 4-(2-chlorophenyl)-1-ethyl-2-methyl-5-oxo-1,4,5,7-tetrahydrofuro[3,4-b]pyridine-3-carboxylate.

9. Medicaments containing at least one compound of the general formula I according to Claim 1.

10. Use of the dihydropyridinelactones according to Claims 1 to 3 for the preparation of medicaments for the combating of diabetes.

## Revendications

1. Lactones de dihydropyridines de formule générale I

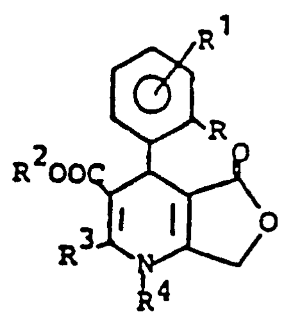

(I)

dans laquelle
R représente un halogène, un groupe alkyle ayant 1 à 10 atomes de carbone ayant 1 à 10 atomes de carbone ou un groupe cyano,
$R_1$ représente l'hydrogène, un halogène ou un groupe alkyle en $C_1$ à $C_{10}$ ou alkoxy en $C_1$ à $C_{10}$,
$R_2$ est un reste hydrocarboné en $C_1$ à $C_{20}$ saturé ou non saturé, à chaîne droite, à chaîne ramifiée ou cyclique, qui peut être interrompu le cas échéant par un ou deux atomes d'oxygène ou -$SO_n$- (n = 0, 1 ou 2) et qui peut être substitué le cas échéant par un ou plusieurs atomes de fluor, chlore, brome ou iode, phényle, -$NO_2$, -O-$NO_2$, trialkylsilyle en $C_3$ à $C_{12}$, -OH, -CN, amino, monoalkylamino en $C_1$ à $C_6$, di(alkyle en $C_1$ à $C_6$)amino, benzyl-(alkyle en $C_1$ à $C_6$)amino,
$R^3$ représente l'hydrogène, un groupe $NH_2$, CHO, CN ou un reste hydrocarboné saturé ou non saturé, à chaîne droite ou à chaîne ramifiée en $C_1$ à $C_8$, qui peut être interrompu dans la chaîne par de l'oxygène, un groupe -NH- ou -N-alkyle (ayant 1 à 6 atomes de carbone),

$R^4$ est un reste alkyle en $C_1$ à $C_{10}$ à chaîne droite ou ramifiée ou un reste alcényle en $C_2$ à $C_{10}$ qui peut être interrompu le cas échéant par un ou deux atomes d'oxygène dans la chaîne alkylique et qui peut être substitué le cas échéant par 1 à 6 atomes de fluor, Cl, Br, I, CN, $NH_2$, OH, phényle, naphtyle, hydroxycarbonyle, alkoxycarbonyle (1 à 10 atomes de carbone dans le reste alkoxy), -CHO, morpholino ou di(alkyle en $C_1$ à $C_4$)amino,

sous la forme d'isomères, de mélanges d'isomères, de racémates et d'antipodes optiques.

2. Lactones de dihydropyridines de formule générale I suivant la revendication 1, dans laquelle

R représente le chlore, le fluor, le brome, un groupe alkyle (1 à 6 atomes de carbone) ou cyano,

$R^1$ est l'hydrogène, le fluor, le chlore, un groupe alkyle (1 à 4 atomes de carbone) ou alkoxy (1 à 4 atomes de carbone),

$R^2$ est un reste alkyle ou alcényle à chaîne droite, à chaîne ramifiée ou cyclique (1 à 12 atomes de carbone), qui peut être interrompu le cas échéant par 1 ou 2 atomes d'oxygène ou de soufre ou groupes -SO-, et qui peut être substitué éventuellement par du fluor, un groupe phényle, $-NO_2$, $-O-NO_2$, -OH, amino, monoalkylamino (1 à 3 atomes de carbone), Cl ou Br,

$R^3$ représente l'hydrogène, un groupe CN ou un reste alkyle ou alcényle à chaîne droite ou ramifiée (1 à 4 atomes de carbone), qui peut être interrompu dans la chaîne par 0 ou un groupe -N(alkyle en $C_1$ à $C_3$),

$R^4$ représente un reste alkyle ou alcényle à chaîne droite ou à chaîne ramifiée (1 à 6 atomes de carbone), qui peut être interrompu le cas échéant par un ou deux atomes d'oxygène dans la chaîne alkylique et qui peut être substitué le cas échéant par 1 à 3 atomes de fluor, Cl, $NH_2$, OH, phényle, hydroxycarbonyle, alkoxycarbonyle (1 à 3 atomes de carbone dans le reste alkoxy), -CHO ou morpholino, sous la forme d'isomères, de mélanges d'isomères, de racémates et d'antipodes optiques.

3. Lactones de dihydropyridines de formule générale I suivant la revendication 1, dans lesquelles

R représente le chlore, le fluor, le brome, un groupe alkyle en $C_1$ à $C_3$,

$R_1$ représente l'hydrogène ou le chlore,

$R_2$ est un groupe alkyle en $C_1$ à $C_6$ qui est interrompu le cas échéant par O, S ou SO, ou un reste phényl-(alkyle en $C_1$ ou $C_2$),

$R^3$ est un reste alkyle en $C_1$ à $C_3$ qui peut être interrompu dans la chaîne par -O- ou $-\underset{CH_3}{\underset{|}{N}}-$,

$R^4$ est un groupe alkyle en $C_1$ à $C_3$, alkylène en $C_2$ ou $C_3$, éventuellement substitué par un radical (alkoxy en $C_1$ à $C_3$)-carbonyle, hydroxycarbonyle.

4. Lactones de dihydropyridines suivant les revendications 1 à 3, destinées à combattre des maladies.

5. Lactones de dihydropyridines suivant les revendications 1 à 3, destinées à combattre le diabète.

6. Procédé de production de lactones de dihydropyridines de formule I

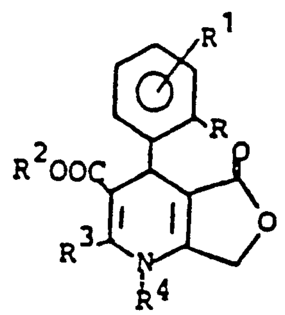

(I)

dans laquelle

R représente un halogène, un groupe alkyle ayant 1 à 10 atomes de carbone ou un groupe cyano,

$R_1$ représente l'hydrogène, un halogène ou un groupe alkyle en $C_1$ à $C_{10}$ ou alkoxy en $C_1$ à $C_{10}$,

$R_2$ est un reste hydrocarboné en $C_1$ à $C_{20}$ saturé ou non saturé, à chaîne droite, à chaîne ramifiée ou cyclique, qui peut être interrompu le cas échéant par un ou deux atomes d'oxygène ou $-SO_n-$ ($n = 0$, 1 ou 2) et qui peut être substitué le cas échéant par un ou plusieurs atomes de fluor, chlore, brome ou iode, phényle, $-NO_2$, $-O-NO_2$, trialkylsilyle en $C_3$ à $C_{12}$, -OH, -CN, amino, monoalkylamino en $C_1$ à $C_6$, di(alkyle en $C_1$ à $C_6$)amino, benzyl-(alkyle en $C_1$ à $C_6$)amino,

$R^3$ représente l'hydrogène, un groupe $NH_2$, CHO, CN ou un reste hydrocarboné saturé ou non saturé à chaîne droite ou à chaîne ramifiée en $C_1$ à $C_8$, qui peut être interrompu dans la chaîne par de l'oxygène, un groupe -NH- ou -N-alkyle (ayant 1 à 6 atomes de carbone),

$R^4$ est un reste alkyle en $C_1$ à $C_{10}$ à chaîne droite ou ramifiée ou un reste alcényle en $C_2$ à $C_{10}$ qui peut être interrompu le cas échéant par un ou deux atomes d'oxygène dans la chaîne alkylique et qui peut être substitué le cas échéant par 1 à 6 atomes de fluor, Cl, Br, I, CN, $NH_2$, OH, aryle, hydroxycarbonyle, alkoxycarbonyle (1 à 10 atomes de carbone dans le reste alkoxy), -CHO, morpholino ou di(alkyle en $C_1$ à $C_4$)amino,

caractérisé en ce que
(A) On élimine des protons de lactones de 1,4-dihydropyridines de formule générale II

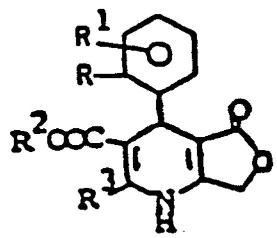

(II),

dans laquelle R, $R^1$, $R^2$, $R^3$ ont la définition indiquée ci-dessus avec des bases dans des solvants inertes et on alkyle le produit avec des composés de formule générale III

$R^4$ - X (III),

dans laquelle $R^4$ a la définition indiquée ci- dessus et
X - représente un groupe facile à éliminer tel que Cl, Br, I, $-OSO_2-R^5$ où $R^5$ est un radical alkyle ou aryle,

ou bien
(B) On fait réagir des aldéhydes de formule générale IV

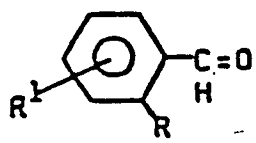

(IV),

dans laquelle R, $R^1$ ont la définition indiquée ci-dessus et des amines de formule générale V ou leurs sels

$H_2N-R^4$ (V)

où $R^4$ a la définition indiquée ci-dessus, avec des composés cétoniques de formule générale VI

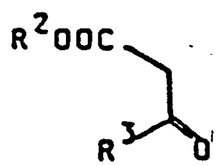

(VI)

dans laquelle $R^2$, $R^3$ ont la définition indiquée ci-dessus, et l'acide tétronique de formule VII

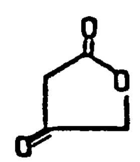

(VII)

le cas échéant en présence d'eau et/ou de solvants organiques inertes,
ou bien
(C) On fait réagir des aldéhydes de formule IV avec des composés cétoniques de formule VI et des énamines de formule générale VIII

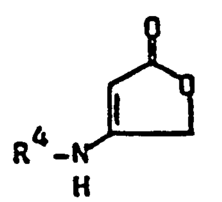

(VIII)

dans laquelle $R^4$ a la définition indiquée ci- dessus, éventuellement en présence d'eau et/ou de solvants organiques inertes,
ou bien

(D) On fait réagir des énamines de formule générale IX

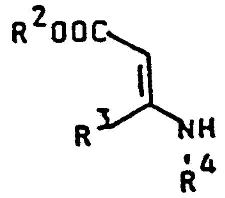

(IX),

dans laquelle $R^2$, $R^3$, $R^4$ ont la définition indiquée ci-dessus, avec des aldéhydes de formule IV et l'acide tétronique de formule VII, éventuellement en présence d'eau et/ou de solvants organiques inertes,

ou bien

(E) On fait réagir des composés ylidéniques de formule générale X

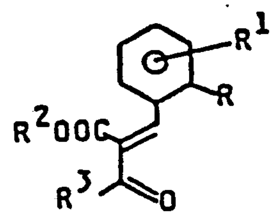

(X),

dans laquelle $R^1$, $R^2$, $R^3$ ont la définition indiquée ci-dessus, avec des énamines de formule VIII, le cas échéant en présence d'eau et/ou de solvants organiques inertes,

ou bien

(F) On fait réagir des énamines de formule IX avec des composés ylidéniques de formule générale XI

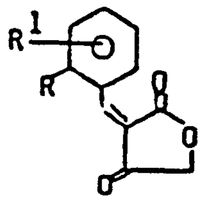

(XI),

dans laquelle R, $R^1$ ont la définition indiquée ci-dessus, éventuellement en présence d'eau ou de solvants organiques inertes,

ou bien

(G) On fait réagir des amines de formule V avec des composés ylidéniques de formule X et l'acide tétronique de formule VII, le cas échéant en présence d'eau et/ou de solvants organiques inertes,

ou bien

(H) On fait réagir des amines de formule V avec des composés cétoniques de formule VI et des composés ylidéniques de formule XI, le cas échéant en présence d'eau et/ou de solvants organiques inertes,

ou bien

(I) On cyclise des dihydropyridines de formule générale XII

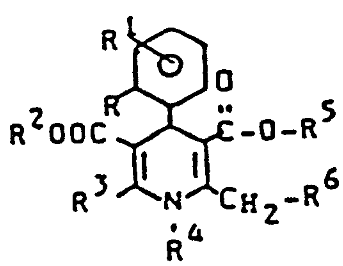

(XII),

dans laquelle R, $R^1$, $R^2$, $R^3$, $R^4$ ont les définitions indiquées ci-dessus, $R^5$ a la même définition que $R^2$,

$R^6$ - représente un halogène, ou bien

- le groupe O-$R^7$ dans lequel $R^7$ désigne un groupe classique protégeant la fonction alcool,

éventuellement en présence d'un solvant organique inerte ainsi que d'une substance auxiliaire appropriée.

7. Procédé suivant la revendication 6 pour la préparation de lactones de dihydropyridines de formule I suivant la revendication 6, dans laquelle

R représente le chlore, le fluor, le brome, un groupe cyano ou alkyle en $C_1$ à $C_3$,
$R_1$ est l'hydrogène ou le chlore,
$R_2$ est un groupe alkyle en $C_1$ à $C_6$ qui est interrompu le cas échéant par O, S ou SO, ou un groupe phényl-(alkyle en $C_1$ ou $C_2$),
$R_3$ est un reste alkyle qui peut être interrompu dans la chaîne par -O- ou $-\underset{CH_3}{\underset{|}{N}}-$,
$R_4$ est un groupe alkyle en $C_1$ à $C_3$, alkylène en $C_2$ ou $C_3$ éventuellement substitué par un radical (alkoxy en $C_1$ à $C_3$)-carbonyle, hydroxycarbonyle.

8. Procédé suivant la revendication 6, pour la préparation de l'ester isopropylique de l'acide 4-(2-chlorophényl)-1-éthyl-2-méthyl-5-oxo-1,4,5,7-tétrahydrofuro[3,4-b]-pyridine-3-carboxylique.

9. Médicament contenant au moins un composé de formule générale I suivant la revendication 1.

10. Utilisation des lactones de dihydropyridines suivant les revendications 1 à 3 pour la préparation de médicaments destinés à la luttre contre le diabète.